# FASCICULE DE BREVET EUROPEEN

(11) **EP 0 830 867 B1**
(45) Date de publication et mention de la délivrance du brevet: **14.12.2005**
(21) Numéro de dépôt: 97420168.3
(22) Date de dépôt: 18.09.1997
(51) Int. Cl.: A61M 1/16, B01D 63/00, A61L 33/00

(54) **Appareil pour le traitement du sang par circulation extracorporelle et procédé de fabrication**
Apparat zur Behandlung des Blutes durch extrakorporalen Blutkreislauf, sowie Verfahren zu seiner Herstellung
Apparatus for treating the blood by extracorporeal circulation and process for manufacturing the same

(30) Priorité: 19.09.1996 FR 9611631; 08.11.1996 FR 9613997
(43) Date de publication de la demande: 25.03.1998
(60) Demande divisionnaire: 05021798.3
(73) Titulaire: Gambro Industries SAS, 69330 Meyzieu (FR)
(72) Inventeur: Thomas, Michel, 69360 Serezin Du Rhone (FR); Valette, Pierre, 69003 Lyon (FR)
(74) Mandataire: Sutto, Luca

(56) Documents cités:
- EP-A- 0 704 223
- US-A- 5 004 548
- US-A- 5 162 102
- US-A- 5 229 172
- US-A- 5 240 994

## Description

La présente invention concerne un procédé de fabrication d'un appareil pour le traitement du sang par circulation extracorporelle et un appareil obtenu de cet procéde.

Des appareils pour le traitement du sang par circulation extracorporelle sont utilisés dans diverses applications médicales ou paramédicales telles que : traitement de l'insuffisance rénale par dialyse ou hémofiltration, plasmaphérèse et aphérèse à visée thérapeutique et non thérapeutique, oxygénation du sang, immunoépuration, etc.

Tous ces appareils ont en commun de comporter un compartiment sang muni de deux accès, dans lequel, lors du traitement considéré, le sang du patient est mis en circulation. Pour ce faire, une canalisation de prélèvement de sang est connectée entre un vaisseau sanguin du patient et un accès, utilisé comme entrée, du compartiment sang; une canalisation de restitution de sang est connectée entre l'autre accès du compartiment sang, utilisé comme sortie, et un vaisseau sanguin du patient; et le sang du patient est mis en circulation dans ce circuit extracorporel bouclé sur le patient, au moyen d'une pompe disposée généralement sur la canalisation de prélèvement.

Le compartiment sang de ces appareils est généralement délimité par une partie des parois d'un boîtier de l'appareil et par une paroi d'un élément actif de l'appareil, au moyen duquel le traitement du sang est effectué. A titre d'exemple, dans un dialyseur à fibres creuses, le compartiment sang est délimité par l'intérieur des fibres d'un faisceau de fibres creuses, constituant une membrane semi-perméable, par la surface extérieure des disques de colle utilisés pour fixer le faisceau de fibres aux deux extrémités d'un boîtier tubulaire de l'appareil, et par deux embouts fixés à chaque extrémité du boîtier.

Tous les matériaux utilisés dans la fabrication de ces appareils sont choisis pour être aussi biocompatibles que possible de façon que les réactions (coagulation, notamment) qui se produisent lorsque le sang entre au contact d'un matériau étranger ne se produisent pas ou à des niveaux relativement bénins.

Il est connu de traiter, dans la masse ou en surface, les matériaux destinés à être en contact avec du sang pour en améliorer la biocompatibilité. Les traitements connus interviennent soit lors de la préparation des solutions de polymères utilisées pour fabriquer telle ou telle partie d'un appareil (traitement massique), soit après que les différentes parties de l'appareil ont été assemblées et avant stérilisation de l'appareil, soit, extemporanément, juste avant l'utilisation de l'appareil.

Le document US 5 162 102 révèle un instrument médical ayant une portion en contact avec le sang formée d'un matériau hydrophobe dans lequel un agent actif en surface, sans danger pour le corps humain, est déposé sur tout ou partie de la portion en contact avec le sang ; par exemple l'agent actif en surface peut être déposé seulement sur la surface interne de l'entrée sang ou de la sortie sang de l'instrument, de façon qu'en introduisant du liquide de rinçage dans l'instrument, l'agent actif en surface soit réparti sur toute la partie de l'instrument en contact avec le sang

Un problème particulièrement ardu à résoudre se pose lorsqu'on cherche à améliorer la biocompatibilité de l'élément actif d'un appareil (membrane de dialyse, par exemple) en respectant les conditions suivantes :
1) le choix de la substance utilisée pour le traitement et les modalités du traitement doivent avoir pour résultat la modification d'un élément actif connu, cette modification ayant pour effet d'améliorer la biocompatibilité de l'élément actif tout en préservant toutes les qualités connues (par exemple, pour une membrane de dialyse/hémofiltration : performances des transferts diffusifs et convectifs, capacité d'adsorption de substances indésirables, etc.);
2) la stérilisation de l'appareil ne doit pas avoir d'influence sur le traitement;
3) le traitement ne doit pas requérir de manipulation particulière de la part de l'utilisateur.

L'invention a pour but de proposer un procédé de fabrication d'un appareil qui satisfasse à ces conditions.

De façon plus spécifique, l'invention a pour but de proposer un procédé de fabrication d'un appareil satisfaisant aux conditions énoncées ci-dessus et dont l'élément actif, avant traitement, présente des charges négatives en surface. Lorsque le sang entre en contact d'une surface négativement chargée, il est le siège d'un phénomène biologique, appelé activation de la phase contact qui se manifeste par la génération de substances actives, la kallicréine et le facteur Xlla, à partir de substances inactives, prékallicréine et facteur XII.

L'activation de la phase contact est bénigne en soi, mais quand elle se produit simultanément à certains facteurs perturbateurs (prise de médicaments hypotenseurs de type IEC par le patient, dilution du sang pénétrant dans l'appareil rempli de solution saline, abaissement concomitant du pH), elle semble à l'origine de réactions indésirables, dites anaphylactoïdes qui se manifestent quelques minutes après le début du traitement par divers symptômes, parmi lesquels la sensation de chaleur généralisée, l'engourdissement des doigts, des lèvres ou de la langue, le halètement, la nausée, l'oedème laryngé. On rappelle que les réactions anaphylactoïdes ne sont pas exclusivement liées à l'utilisation d'appareils médicaux dont le compartiment sang a une surface interne chargée négativement. Ces réactions ont été observées avec des échangeurs ayant des membranes de différentes compositions chimiques, tantôt lors d'une première utilisation, tantôt après plusieurs utilisations lorsque les échangeurs, au lieu d'être jetés après un usage unique, sont réutilisés de multiples fois et sont recyclés après chaque usage. Comme exemple d'échangeurs dont une première utilisation s'est accompagnée d'une réaction indésirable, on peut citer les dialyseurs ayant une membrane de polyméthylméthacrylate et de polyacrylonitrile. Des réactions associées à la réutilisation des dialyseurs à membrane d'acétate de cellulose et de polysulfone ont été également bien documentées (voir "Anaphylactoid reactions associated with reuse of hollow-fiber hemodialyzers and ACE inhibitors" in Kidney International, vol. 42 (1992), pp. 1232-1237).

Pour atteindre ce but, on prévoit, conformément à l'invention un procédé selon la revendication 1.

Au sen de l'invention, l'aptitude à la dissolution dans une solution aqueuse, qui est une caractéristique de la substance, doit se comprendre en fonction de l'objectif à atteindre, qui est qu'à l'issue du procédé, au moins une couche moléculaire de la substance est liée à la surface interne à traiter du compartiment sang. En outre, lorsqu'il serait indésirable que la substance soit introduite dans le sang du patient, il faut qu'à l'issue du procédé toute la substance ait été dissoute et qu'aucun résidu ne demeure dans le compartiment sang. En d'autre termes, cette aptitude comporte à la fois la capacité physique à se dissoudre dans une solution aqueuse et une vitesse de dissolution, qui dépend de plusieurs paramètres : volume de solution aqueuse utilisé, température de la solution aqueuse, débit de la solution aqueuse dans le compartiment sang. Si l'on fixe ces paramètres en décidant par exemple que la dernière étape du procédé sera réalisée immédiatement avant l'utilisation de l'appareil lors de la procédure d'amorçage de l'appareil, alors la substance doit être choisie, entre autres critères, de façon que l'objectif de l'invention soit atteint en faisant circuler dans le compartiment sang, par exemple, deux litres de sérum physiologique, à température ambiante (20°C à 24°C), à un débit de 200 ml/min (conditions classiques de l'amorçage d'un dialyseur dans un centre de dialyse).

Le procédé présente deux avantages majeurs : d'une part, la surface modifiée par la substance n'est obtenue qu'après stérilisation de l'appareil de sorte que cette surface modifiée, biocompatible, ne risque pas d'être endommagée par une stérilisation très énergétique, comme la stérilisation par irradiation γ; d'autre part, lorsque la solution aqueuse utilisée est la solution d'amorçage de l'échangeur, la mise en oeuvre de l'appareil par l'utilisateur est exactement identique à celle de tout appareil du même type.

Selon une variante de l'invention, dans un appareil dont le compartiment sang a deux accès symétriques, la quantité déterminée de substance est déposée au niveau de chaque accès de façon que, quel que soit le sens de la circulation ultérieure de la solution aqueuse dans le compartiment sang, une couche moléculaire de la substance soit formée sur la surface interne à traiter.

Ce procédé présente l'avantage d'être très facile à mettre en oeuvre au plan industriel.

L'invention a également pour objet un appareil pour le traitement du sang ou du plasma selon la revendication 8.

Selon une variante de l'invention, la surface à traiter présente des charges négatives et la substance est cationique de sorte qu'une couche moléculaire de la substance masque les charges négatives de la surface.

Dans un mode de réalisation de l'invention, l'appareil est un hémodialyseur/hémofiltre muni d'une membrane fabriquée à partir d'un copolymère d'acrylonitrile et de méthallyle sulfonate de sodium (membrane connue sous le nom commercial AN69). La substance choisie pour améliorer la biocompatibilité de cette membrane est de la polyéthylènimine (PEI) ayant une masse moléculaire (mesurée par diffusion de lumière) comprise entre environ 10 et environ 2000 k µ (µ = unité de masse atomique = Dalton). La quantité de PEI (masse moléculaire, 25 k µ) déposée au niveau d'un ou de chaque accès sang est comprise de préférence entre environ 5 et environ 10 mg par m² de membrane destinée à être au contact du sang.

Cet appareil n'active pas la phase contact et a les mêmes performances qu'un hémodialyseur/hémofiltre non modifié.

D'autres caractéristiques et avantages de l'invention apparaîtront à la lecture de la description qui suit. On se reportera aux dessins annexés sur lesquels :
la figure 1 représente une vue en coupe longitudinale schématique d'un dialyseur à fibres creuses selon l'invention;
la figure 2 représente l'effet de la quantité de PEI utilisée pour traiter de la membrane AN69 sur le potentiel électrique de surface de cette, membrane;

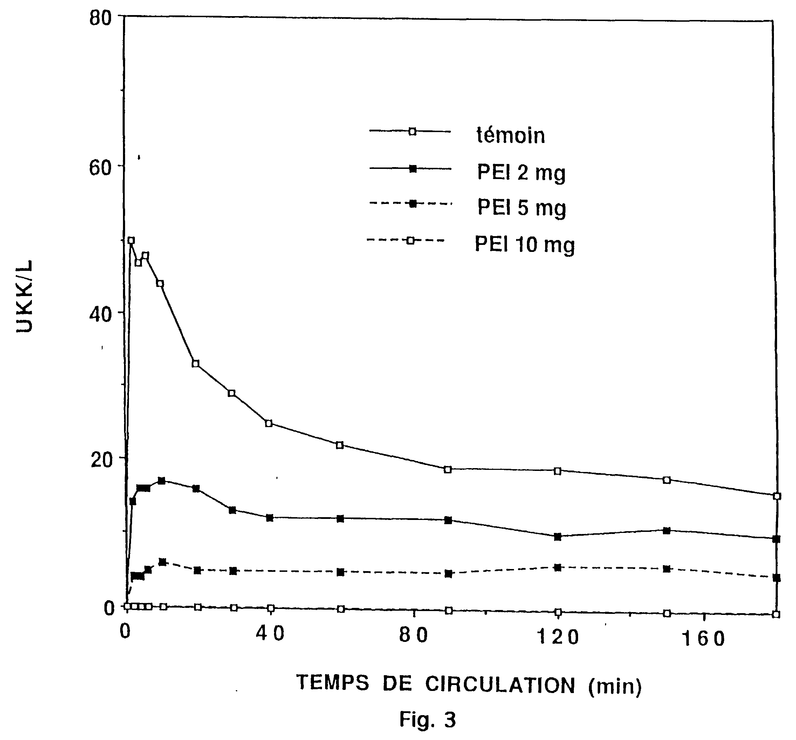

la figure 3 représente l'effet de la quantité de PEI (masse moléculaire : 25 k µ) utilisée pour traiter de la membrane AN69 sur l'activation de la phase contact;
la figure 4 représente l'effet de la quantité de PEI (masse moléculaire : 750 k µ) utilisée pour traiter de la membrane AN69 sur l'activation de la phase contact;
la figure 5 représente la cinétique d'adsorption du Cytochrome C sur une membrane en AN69 classique et sur une membrane en AN69 à laquelle de la PEI a été liée;
la figure 6 représente le niveau d'activation de la phase contact par une membrane expérimentale dépourvue de PEI et par la même membrane à laquelle de la PEI a été liée;
la figure 7 représente le niveau d'activation de la phase contact par une membrane à base de polyacrylonitrile dépourvue de PEI et par la même membrane à laquelle de la PEI a été liée;
la figure 8 représente le niveau d'activation de la phase contact par une membrane en AN69 dépourvue de DEAE dextran et par la même membrane à laquelle du DEAE dextran a été lié; et

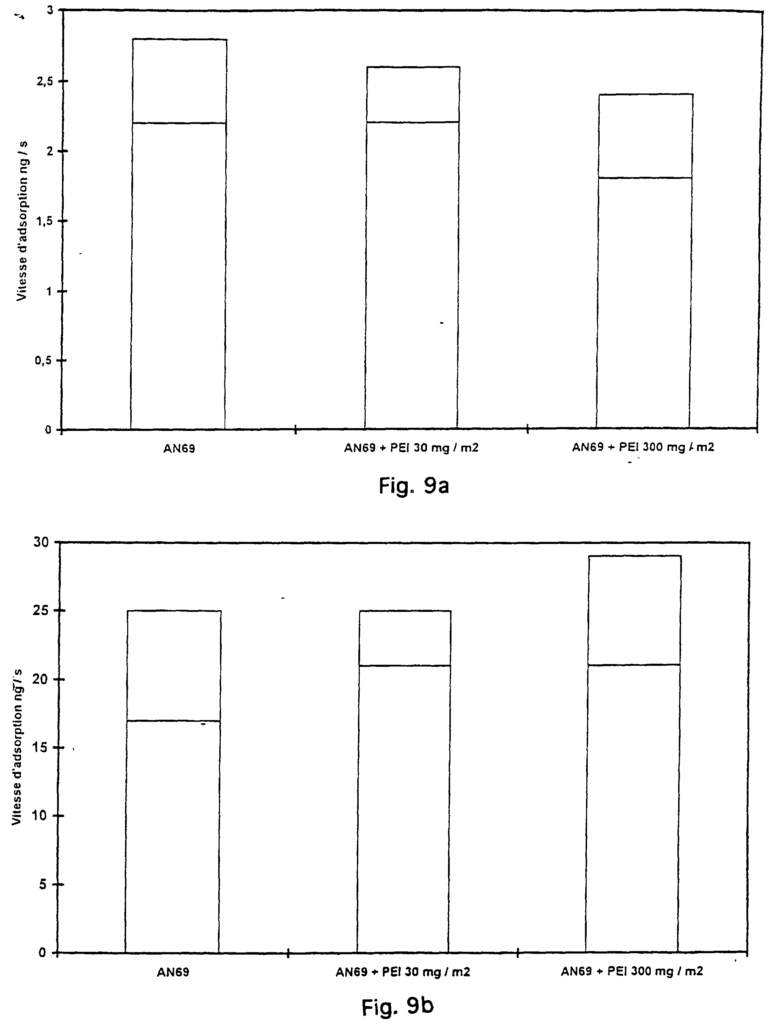

les figures 9a et 9b illustrent l'hémocompatibilité d'une membrane de dialyse selon l'invention.

Pour illustrer l'invention, on décrira un type particulier d'appareil pour le traitement extracorporel du sang qui est utilisé pour pallier l'insuffisance rénale.

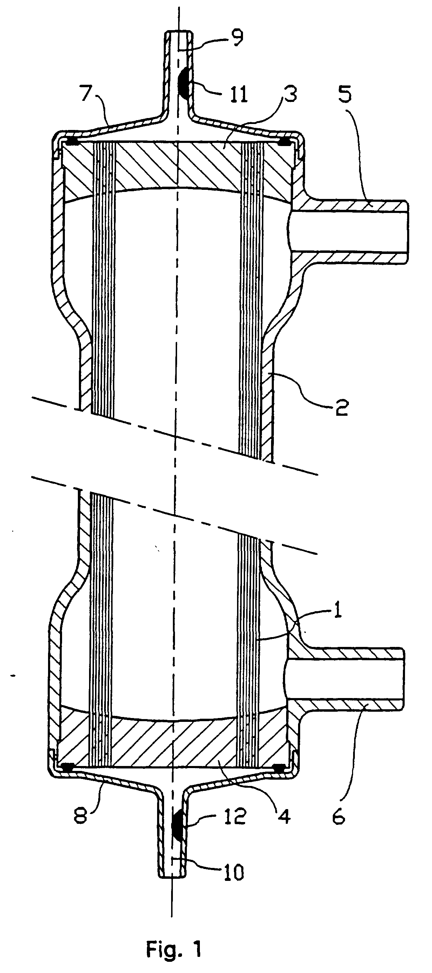

Un hémodialyseur/hémofiltre comprend, de façon classique, deux compartiments séparés par une membrane semi-perméable. Un premier compartiment est destiné à être relié au moyen d'une canalisation de prélèvement et d'une canalisation de restitution au circuit vasculaire du patient, tandis que le second compartiment a une entrée éventuellement reliée à une source de liquide de dialyse (traitement par hémodialyse et hémodiafiltration) et une sortie reliée à une évacuation de liquide usé (dialysat usé et/ou ultrafiltrat). La membrane est choisie pour permettre les transferts diffusifs et/ou convectifs des déchets du métabolisme, à partir du compartiment sang vers le compartiment pour liquide usé. La membrane peut être fabriquée sous la forme d'une membrane plane ou d'un faisceau de fibres creuses. Un dialyseur à membrane plane comprend une bande de membrane plane pliée en accordéon, une plaque intercalaire étant introduite dans tous les plis ouvrant d'un même côté. Comme on peut le voir sur la figure 1, un dialyseur à fibres creuses comprend un faisceau de fibres creuses 1, qui est disposé dans un boîtier tubulaire 2 où il est assujetti à ses deux extrémités par un disque de colle 3, 4. Outre de lier les fibres les unes aux autres, les disques de colle 3, 4 ont pour fonction de délimiter dans le boîtier tubulaire 2 un compartiment étanche auquel deux tubulures 5, 6, perpendiculaires à l'axe du boîtier 2, donnent accès. A chaque extrémité du boîtier 2 est fixé un embout 7, 8 comprenant une tubulure d'accès axiale 9, 10. Les deux tubulures 9, 10 sont symétriques. Le compartiment sang de cet appareil est constitué par l'espace intérieur délimité entre chaque disque de colle 3, 4 et l'embout 8, 9 fermant l'extrémité correspondante du boîtier tubulaire 2, et par l'intérieur des fibres creuses.

Conformément à l'invention, pour améliorer la biocompatibilité de cet appareil en modifiant une surface interne du compartiment sang, on dépose dans chaque embout 8, 9, après que l'appareil a été assemblé comme représenté sur la figure 1, une quantité déterminée d'une substance soluble dans une solution aqueuse et apte à modifier la surface concernée de la façon souhaitée. La quantité de substance est choisie de façon que, après circulation d'une quantité déterminée de solution aqueuse dans le compartiment sang, au moins une couche moléculaire de la substance recouvre la surface dont la biocompatibilité est à améliorer. La quantité de substance est déposée sous la forme d'une goutte 11, 12 au moyen d'un dispositif d'injection classique. La goutte est formée soit d'un gel de la substance soit d'un matériau matrice dans lequel la substance est incorporée.

Après que le dépôt de la substance a été effectuée, les tubulures d'accès 5, 6, 9, 10 sont munies de bouchons et l'appareil peut être stérilisé, par exemple au moyen d'oxyde d'éthylène ou par irradiation γ.

L'utilisation de tout appareil pour le traitement du sang ou du plasma par circulation extracorporelle comprend une phase préliminaire d'amorçage, au cours de laquelle le compartiment sang est rincé et rempli d'une solution aqueuse stérile.

Conformément à l'invention, on met à profit cette phase de préparation de l'appareil pour dissoudre la substance de traitement et la porter au contact de la surface à traiter : l'utilisateur, avant le début de la séance de dialyse proprement dite, connecte l'une des tubulures d'accès 9 (10) du circuit sang à une poche de solution stérile, connecte l'autre tubulure d'accès 10 (9) à une poche de recueil vide, et provoque la circulation de la solution stérile dans le compartiment sang, le cas échéant au moyen de la pompe à sang de la machine de dialyse. La solution stérile dissout la substance et la porte au contact de la surface à traiter où elle se lie, par exemple par liaison ionique ou covalente.

Dans l'exemple de réalisation qui vient d'être décrit, c'est parce que les deux tubulures d'accès 9, 10 au compartiment sang sont symétriques et pour ne pas avoir à imposer à l'utilisateur un sens de circulation de la solution saline lors de l'amorçage de l'hémodialyseur, que l'on a déposé une quantité de substance appropriée (gouttes 11, 12) dans chacun des embouts. La substance qui est déposée dans l'embout aval lors de l'amorçage n'est donc pas utilisée pour le traitement de la surface dont la biocompatibilité est à améliorer. Naturellement, moyennant de repérer convenablement l'embout auquel connecter la poche de solution saline pour l'amorçage, il est possible de ne déposer la goutte de la substance de traitement que dans un embout 7, 8.

Selon une variante de l'invention, au lieu de fabriquer un appareil de traitement de sang selon le procédé décrit plus haut, où une goutte de substance appropriée est déposée dans au moins l'une des tubulures d'accès 9, 10 au compartiment sang, on fabrique un raccord comportant une partie femelle complémentaire de ces tubulures 9, 10 ainsi qu'une partie mâle complémentaire de l'élément de connexion femelle équipant l'extrémité des canalisations utilisées pour relier un patient à un appareil de traitement du sang. Ce raccord est obtenu par moulage ou extrusion d'une matière plastique apte au contact avec le sang. On dépose à l'intérieur de ce raccord une goutte de la substance destinée à améliorer la biocompatibilité d'un appareil de traitement du sang. Le raccord est ensuite placé dans un emballage susceptible de constituer une barrière stérile puis, l'ensemble est stérilisé par irradiation γ. Le raccord est monté sur un appareil de traitement du sang classique avant la phase préliminaire d'amorçage de l'appareil décrite plus haut.

Dans un mode de réalisation de l'invention, la partie de l'hémodialyseur dont la biocompatibilité est à améliorer est une membrane semi-perméable dont la surface présente des charges négatives. Le but de l'amélioration est d'empêcher l'activation de la phase contact ou de la neutraliser. Conformément à l'invention, une substance appropriée présente les caractéristiques suivantes :
1 - elle doit être cationique de façon à se lier par liaison ionique à la membrane et à en masquer les charges négatives;
2 - elle doit être soluble dans l'eau de façon à pouvoir être dissoute par la solution aqueuse utilisée pour l'amorçage de l'appareil;
3 - elle ne doit pas être toxique;
4 - elle doit être macromoléculaire et sa taille doit être choisie de façon qu'une macromolécule ne pénètre pas dans les pores de la membrane (à titre d'exemple, pour la membrane en AN69, cette taille doit être de au moins 10 k µ). En cours d'utilisation, une macromolécule fixée à la membrane en sera difficilement délogée par une protéine du sang. Aussi, une macromolécule, à qui sa taille interdit de pénétrer dans les cellules biologiques, est à priori moins toxique qu'une molécule que ses dimensions autorisent à pénétrer dans une cellule ;
5 - elle doit supporter le cas échéant, même si elle en est partiellement affectée, une stérilisation énergétique, du type irradiation γ. En d'autres termes, au moins une partie des molécules doit rester intacte et pouvoir se lier de la façon souhaitée à la membrane. Par ailleurs la substance irradiée ne doit pas devenir toxique ;
6 - elle ne doit pas, une fois liée à la membrane, en altérer de façon significative les caractéristiques (hémocompatibilité, capacité de transfert diffusif et convectif, capacité d'adsorption protéique).

A titre d'exemple, on a découvert, conformément à l'invention, que la PEI de masse moléculaire comprise entre 10 et 2000 k µ qui se présente sous la forme d'un gel soluble dans l'eau, est une substance tout à fait appropriée à la modification de la membrane connue sous le nom commercial AN69, dont la surface présente des charges négatives. En particulier, des essais *in vitro* ont montré que le seuil de toxicité potentiel de la PEI irradiée correspond à l'injection d'une quantité de PEI nécessaire pour élever la concentration d'un liquide intérieur (plasma) à 0,4 mg/ml (à titre de comparaison, si la quantité de PEI suffisante pour traiter un dialyseur classique, soit 10 mg, était injectée dans le sang d'un adulte, la concentration du plasma en PEI serait de l'ordre de 0, 002 mg/ml).

Une autre substance appropriée à la modification de la membrane connue sous le nom commercial AN69 est le diéthylaminoéthyl dextran (DEAE dextran) de masse moléculaire moyenne de 500 k Dalton. Comme cette substance, à la différence de la PEI, n'est pas disponible sous la forme d'un gel soluble dans l'eau mais d'une poudre, il faut, pour pouvoir l'utiliser conformément à l'invention, l'incorporer dans un gel d'un matériau matrice neutre. A titre d'exemple, on peut utiliser à cette fin un gel de carboxyméthyl-cellulose.

On rappelle brièvement les principales étapes de la fabrication d'une fibre creuse en AN69. Une solution de polymère est préparée, contenant 35 % en poids d'un copolymère d'acrylonitrile et de méthallyle sulfonate de sodium, 52 % en poids de diméthylformamide (DMF) et 13 % en poids de glycérol. La solution de polymère est chauffée à 130° C et est extrudée dans une filière ayant deux buses concentriques, de l'azote étant injecté dans la buse interne pour former la lumière de la fibre creuse. Au contact de l'air ambiant (environ 20-25 °C), la fibre de gel thermoréversible sortant de la filière est le siège d'une inversion de phase thermique. La fibre est ensuite reçue dans un bain d'eau dans lequel le solvant (DMF) dans la fibre est remplacé par de l'eau. La fibre est ensuite plongée dans de l'eau chaude à 95 ° C où elle est étirée de l'ordre de quatre fois. Suit une phase de stabilisation dans de l'eau chaude à 95 ° C. Enfin la fibre est glycérinée avec un mélange eau/glycérol.

La fabrication d'une membrane plane à partir d'AN69 comporte les étapes suivantes : une solution de polymère est préparée, contenant 21 % en poids d'un copolymère d'acrylonitrile et de méthallyle sulfonate de sodium, et 79 % en poids de diméthylformamide (DMF). Après filtration et dégazage, cette solution de polymère est extrudée au moyen d'une filière en forme de fente sur un cylindre rotatif chauffé à 80° C. Une partie du DMF est évaporé. Le film obtenu est étiré de l'ordre de trois fois et demi dans de l'eau chaude à 95°C. Suit une phase de stabilisation dans de l'eau chaude à 95°C. Enfin la membrane est glycérinée dans un mélange eau/glycérol.

### Exemple 1

Un dialyseur comprenant environ 8500 fibres creuses AN69 a été assemblé. Chaque fibre a les dimensions suivantes : diamètre interne : 240 µm; épaisseur de la paroi : 50µm; longueur : 0,225 m. La surface de la membrane destinée à venir au contact du sang est de environ 1,44 m². Une goutte de 10 mg de PEI (LUPASOL WF, de BASF; masse moléculaire : 25 k µ) a été déposée dans chaque tubulure d'accès au compartiment sang, après quoi les tubulures d'accès aux deux compartiments du dialyseur ont été obturées avec des bouchons spéciaux et le dialyseur a été stérilisé à l'oxyde d'éthylène. Deux litres de sérum physiologique stérile (solution de chlorure de sodium à 0,9 g/l) à température ambiante (22°C) ont été mis en circulation à un débit de 200 ml/min dans le compartiment sang de ce dialyseur. Le sérum physiologique a dissout la goutte de gel de PEI et les molécules de PEI mises en circulation dans le dialyseur se sont liées, par liaison ionique, aux groupes méthallyle sulfonate de sodium présents à la surface de la membrane.

Pour mesurer que le dialyseur ainsi fabriqué et préparé à l'emploi avait le niveau de biocompatibilité assigné comme but à l'invention, on a soumis ce dialyseur au test suivant : un liquide biologique a été préparé, propre à stimuler la production de kallicréines au contact d'une membrane chargée négativement en surface. Le liquide biologique utilisé pour l'essai était constitué de plasma humain pauvre en plaquettes, dilué à 5 % dans du sérum physiologique additionné de citrate (on note que les conditions du test utilisé sont éloignées des conditions d'utilisation d'un appareil pour circulation extracorporelle de sang : le taux de dilution est très élevé, le liquide choisi est du plasma et non du sang, le plasma est additionné de citrate, donc acidifié, alors qu'en dialyse, l'anticoagulant utilisé est de l'héparine. Ces conditions de test sont choisies à dessein car elles stimulent et amplifient l'activation de la phase contact). Un litre et demi de ce liquide ont été mis en circulation en circuit fermé dans le compartiment sang du dialyseur à un débit de 100 ml/min pendant six heures. Les kallicréines plasmatiques ont été dosées dans des échantillons de liquide prélevés à intervalle de temps au moyen d'un test chromogénique classique, à partir du substrat S 2302 de la société BIOGENIC. Le résultat des dosages montre sans équivoque que le dialyseur fabriqué conformément à l'invention ne provoque pas l'élévation du taux de kallicréines dans un plasma dilué.

On précise que, compte tenu de la sensibilité du test chromogénique utilisé, on considère qu'il n'y a pas d'élévation significative du taux de kallicréines si la concentration en kallicréines reste en deçà d'environ 10 unités par litre.

On souligne que, outre son efficacité en ce qui concerne l'amélioration de la biocompatibilité du dialyseur décrit ci-dessus, ce procédé de fabrication présente un intérêt majeur, même lorsque la stérilisation par l'oxyde d'éthylène permettrait en principe de fixer la PEI sur la membrane avant l'étape de stérilisation. En effet, compte tenu que les fibres en AN69 sont glycérinés, si l'on entreprenait de les traiter avec de la PEI avant la stérilisation du dialyseur, il faudrait :
1 - déglycériner les fibres en rinçant le dialyseur avec une solution aqueuse;
2 - faire circuler dans le compartiment sang une solution de PEI;
3 - reglycériner les fibres pour en chasser l'eau, l'oxyde d'éthylène n'ayant pas d'action sur un produit humide; et
4 - purger les fibres de l'excès de glycérol.

Outre que la quatrième étape serait difficile voire impossible à réaliser, on comprend qu'ajouter à un procédé de fabrication industriel quatre étapes supplémentaires en rendrait le coût prohibitif. En résumé, le procédé selon l'invention, selon lequel le déglycérinage des fibres et leur traitement par la PEI se fait simultanément lors de l'amorçage du dialyseur, rend industrialisable l'amélioration de la biocompatibilité d'un dialyseur dont la membrane est en AN69.

### Exemple 2

Un dialyseur à membrane plane en AN69 a été assemblé. La membrane a une épaisseur de environ 20 µm. La surface de la membrane destinée à venir au contact du sang est de environ 1,50 m². Une goutte de 10 mg de PEI (LUPASOL WF, 25 k µ) a été déposée dans chaque tubulure d'accès au compartiment sang, après quoi les tubulures d'accès aux deux compartiments du dialyseur ont été obturées et le dialyseur a été stérilisé par irradiation γ (36 KGy). Deux litres de sérum physiologique stérile à température ambiante (environ 22°C) ont été mis en circulation dans le compartiment sang de ce dialyseur. Le sérum physiologique a dissout la goutte de gel de PEI et les molécules de PEI mises en circulation dans le dialyseur se sont liées aux groupes méthallyle sulfonate de sodium présents à la surface de la membrane.

Le dialyseur est alors soumis au test in vitro décrit dans l'exemple 1, destiné à mesurer si la membrane AN69 revêtue d'une monocouche de PEI active la phase contact. Comme dans le cas du dialyseur à fibres creuses, le résultat du test est négatif.

### Exemple 3

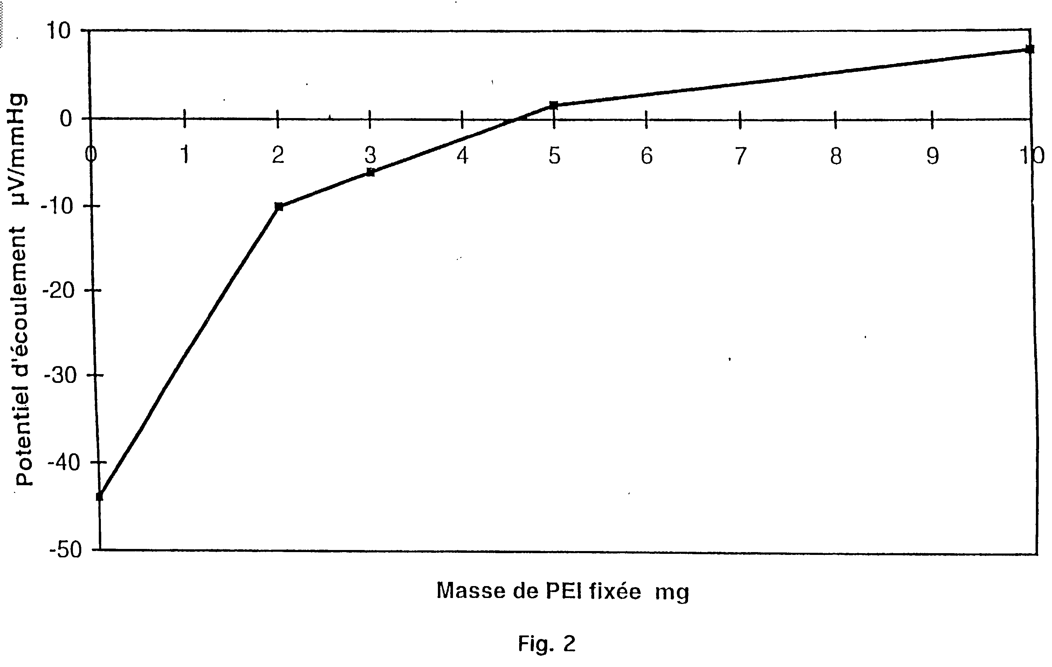

Le graphe de la figure 2 montre le résultat d'essais in vitro effectués sur un dialyseur à membrane plane du type de l'exemple 2, destinés à déterminer l'effet de la quantité de PEI (LUPASOL WF, 25 k µ) utilisée par dialyseur sur la charge électrique de surface d'une membrane en AN69. La charge électrique de surface est évaluée par l'intermédiaire de la mesure du potentiel d'écoulement, tel que défini ci-dessous : on part de ce qu'une solution d'électrolyte circulant dans le compartiment d'un dialyseur engendre une différence de potentiel ΔE proportionnelle à la perte de charge ΔP créée par la solution d'électrolyte entre l'entrée et la sortie du dialyseur. Après que le dialyseur a été rincé et que sa membrane a donc été revêtue de PEI, une solution de chlorure de sodium (10⁻² M) a été mise en circulation dans le compartiment sang, et l'on a mesuré ΔE et ΔP au niveau des accès au compartiment sang, au moyen d'électrodes Ag/AgCl et de capteurs de pression. Le rapport ΔE/ΔP est appelé potentiel d'écoulement de l'électrolyte dans le dialyseur et ce rapport est caractéristique de la charge de la surface de la membrane.

Sur ce diagramme, on voit que environ 5 mg de PEI sont suffisants pour rendre électriquement neutre la surface de la membrane.

### Exemple 4

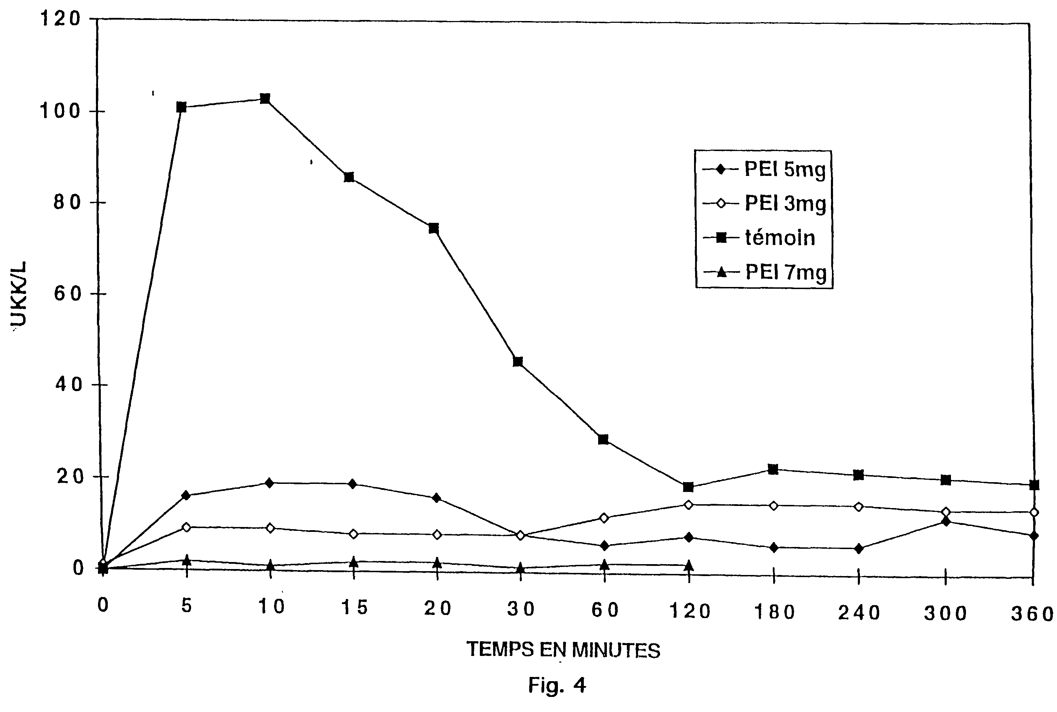

Le graphe de la figure 4 montre le résultat d'essais in vitro effectués sur un dialyseur à membrane plane du type de l'exemple 2, destinés à déterminer l'effet de la quantité de PEI (LUPASOL WF, 25 k µ) utilisée par dialyseur sur l'activation de la phase contact: L'activation de la phase contact est évaluée par le biais de la génération de kallicréines selon le test décrit à l'exemple 1. Sur ce diagramme, on voit que 10 mg de PEI (masse moléculaire, 25 k Dalton) suffisent à supprimer complètement l'activation de la phase contact.

### Exemple 5

Le graphe de la figure 4 montre le résultat d'essais in vitro effectués sur un dialyseur à membrane plane du type de l'exemple 2, destinés à déterminer l'effet de la quantité de PEI (LUPASOL P, 750 k µ) utilisée par dialyseur sur l'activation de la phase contact. L'activation de la phase contact est évaluée par le biais de la génération de kallicréines selon le test décrit à l'exemple 1. Sur ce diagramme, on voit que 7 mg de PEI (masse moléculaire, 750 k Dalton) suffisent à supprimer complètement l'activation de la phase contact.

### Exemple 6

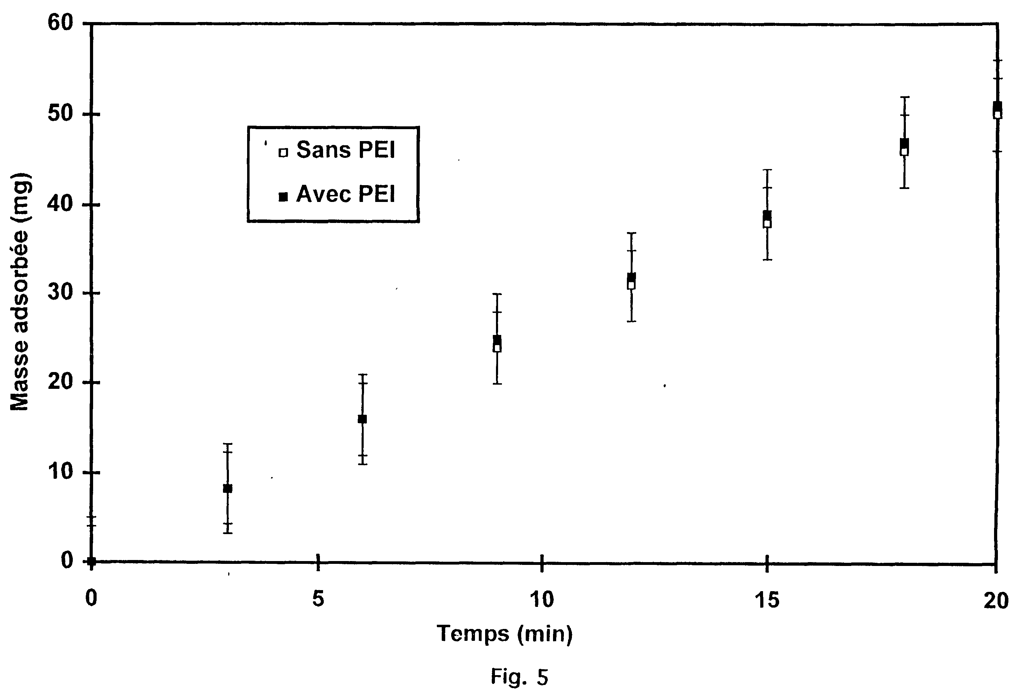

On a vérifié in vitro, en utilisant le cytochrome C comme molécule de test, que la capacité d'adsorption de protéines dans la masse de la membrane reste inchangée après traitement de la membrane avec de la PEI (LUPASOL WF, 25 k µ) à raison de 10 mg par dialyseur à membrane plane en AN69, ayant une surface de environ 1,25 m². Pour effectuer cette vérification, on a pratiqué l'essai suivant sur deux dialyseurs à membrane plane en AN69 (surface de environ 1,25 m²) dont l'un avait été traité avec de la PEI et l'autre pas : six litres d'une solution de cytochrome C à une concentration de 20 mg/l dans un milieu tampon phosphate de pH 7,4 ont été mis en circulation, en circuit ouvert, à un débit de 300 ml/min, dans le compartiment sang de chaque dialyseur. On a mesuré à intervalles de temps réguliers la concentration du cytochrome C dans la solution sortant du dialyseur et on a calculé la quantité cumulée de cytochrome C adsorbée. La figure 5 montre que le résultat des mesures est le même pour les deux dialyseurs.

### Exemple 7

On a également vérifié in vitro que la liaison de la PEI sur la membrane était totalement irréversible et que de la PEI n'était pas relarguée dans un liquide mis en circulation dans le compartiment sang du dialyseur. Pour effectuer cette vérification, on a effectué deux tests indépendants :

1 ° test : on a mesuré le potentiel d'écoulement d'une solution de chlorure de sodium (selon la méthode de l'exemple 3) dans le compartiment sang d'un dialyseur du type de celui qui a été décrit à l'exemple 2. On a fait ensuite circuler en circuit ouvert du sérum physiologique à 37°C, à un débit de 300 ml/min, pendant 5 heures, dans le compartiment sang du dialyseur. On a mesuré alors à nouveau le potentiel d'écoulement d'une solution chlorure de sodium et on a comparé le résultat des deux mesures. De leur identité, on a déduit que la liaison entre la membrane en AN69 et la PEI est durable.

2° test : on a marqué de la PEI (LUPASOL P, 750 k µ) avec une molécule possédant un isotope radioactif (N-succimidyl[2-3 ³H] propionate) en pratiquant de la façon suivante : on a fait réagir de la PEI avec du N-succimidyl[2-3 ³H] propionate pour obtenir de la PEI ³H (PEI marquée) que l'on a fait précipiter ensuite avec de l'acide sulfosalicylique pour séparer la PEI marquée de l'excédent de N-succimidyl[2-3 ³H] propionate ; on a ensuite soumis le précipité à des lavages successifs à l'eau pour éliminer l'excédent de N-succimidyl[2-3 ³H] propionate et obtenir de la PEI marquée ayant une activité spécifique de 1,52 mCi/g (ce qui correspond à un taux moyen de substitution de 11,7 mmol de propionate 3H par µmol de PEI.

Un dialyseur comprenant environ 8500 fibres creuses AN69 a été fabriqué conformément au procédé décrit au premier paragraphe de l'exemple 1, à cette différence près que c'est une goutte de 10 mg de PEI marquée qui a été déposée dans chaque tubulure d'accès au compartiment sang du dialyseur et non pas de la PEI standard (LUPASOL WF, de BASF; masse moléculaire : 25 k µ).

On a fait circuler en circuit fermé pendant quatre heures dans le compartiment sang de ce dialyseur 0,5 l de sang total humain hépariné à 3 Ul/ml (héparine standard commercialisée par PAN PHARMA). On a prélevé toute les demi-heures un échantillon du sang mis en circulation dans le dialyseur et on en a mesuré la radioactivité. Aucune désorption de PEI marquée n'a été détectée dans aucun échantillon (compte tenu des moyens de mesure utilisés, on considère que la limite de détection était de 0,4µg/ml).

### Exemple 8

On a fabriqué une membrane expérimentale M sous la forme d'une fibre creuse selon les étapes suivantes. Une solution de polymère a été préparée, contenant 28 % en poids d'un copolymère d'acrylonitrile et d'acétate de vinyle, 51 % en poids de diméthylformamide (DMF) et 21 % en poids de glycérol. La solution de polymère a été chauffée à 120° C et elle a été extrudée dans une filière ayant deux buses concentriques, de l'azote étant injecté dans la buse interne pour former la lumière de la fibre creuse. Au contact de l'air ambiant (environ 20-25 °C), la fibre de gel thermoréversible sortant de la filière a été le siège d'une inversion de phase thermique. La fibre a été ensuite reçue dans un bain d'eau dans lequel le solvant (DMF) dans la fibre a été remplacé par de l'eau. La fibre a ensuite été plongée dans de l'eau chaude à 40°C où elle a été étirée de l'ordre de deux fois. La fibre résultante a ensuite été stabilisée dans de l'eau chaude à 60°C. Enfin la fibre a été glycérinée dans un mélange eau/glycérol.

Un dialyseur comprenant environ 10000 fibres creuses M a été assemblé. Chaque, fibre a les dimensions suivantes : diamètre interne : 190 µm; épaisseur de la paroi : 50µm; longueur : 0,225 m. La surface de la membrane destinée à venir au contact du sang est de environ 1,34 m². Une goutte de 12 mg de PEI (LUPASOL WF; masse moléculaire: 25 k µ) a été déposée dans chaque tubulure d'accès au compartiment sang. Deux litres de sérum physiologique stérile (solution de chlorure de sodium à 0,9 g/l) à température ambiante (22°C) ont été mis en circulation à un débit de 200 ml/min dans le compartiment sang de ce dialyseur. Le sérum physiologique a dissout la goutte de gel de PEI et les molécules de PEI mises en circulation dans le dialyseur se sont liées, par liaison ionique, à la surface de la membrane.

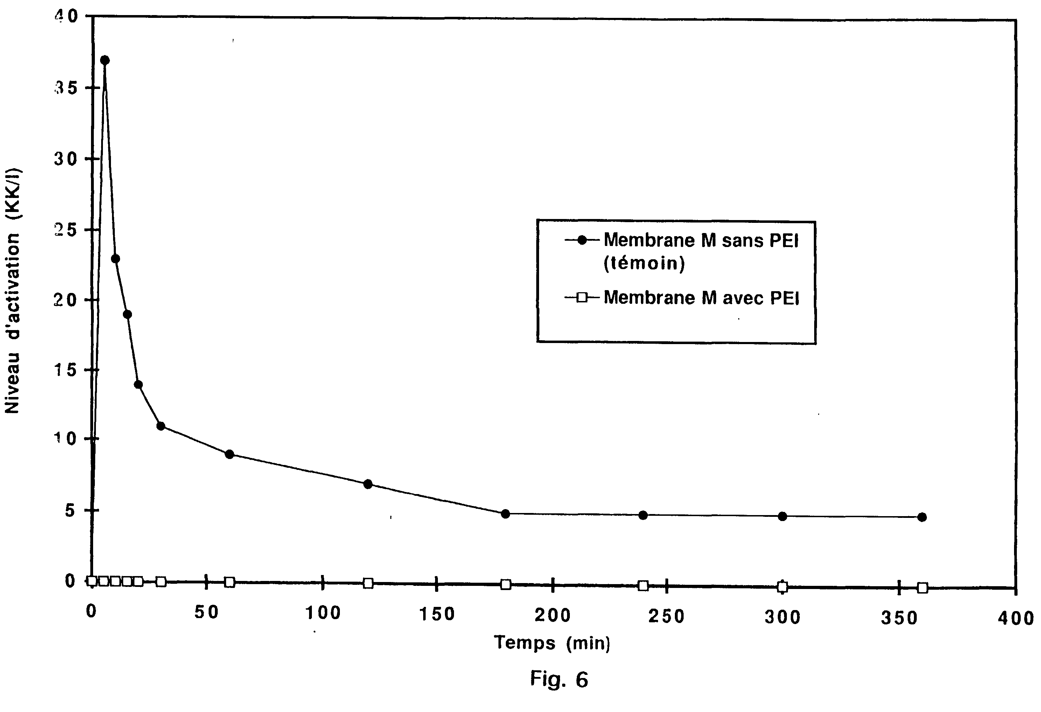

Le dialyseur a alors été soumis au test in vitro décrit dans l'exemple 1, destiné à mesurer si la membrane M revêtue d'une monocouche de PEI active la phase contact. Comme dans le cas du dialyseur à fibres creuses, le résultat du test est négatif. La figure 6 représente le niveau d'activation causé par ce dialyseur et le niveau d'activation causé par un dialyseur de même fabrication mais dont la membrane n'a pas été revêtue de PEI.

### Exemple 9

On a vérifié l'efficacité de l'invention sur un dialyseur à fibres creuses équipé d'une membrane à base de polyacrylonitrile disponible dans le commerce, à savoir un dialyseur de modèle PAN 13 DX (surface utile de la membrane : 1,3 m²) fabriqué par la société ASAHI. Ce dialyseur a été traité conformément au procédé décrit au premier paragraphe de l'exemple 1, à cette différence près que c'est une goutte de 10 mg de PEI de masse moléculaire 750 k µ (LUPASOL P, de BASF) qui a été déposée dans la tubulure d'entrée au compartiment sang du dialyseur et non pas de la PEI de masse moléculaire 25 k µ (LUPASOL WF, de BASF).

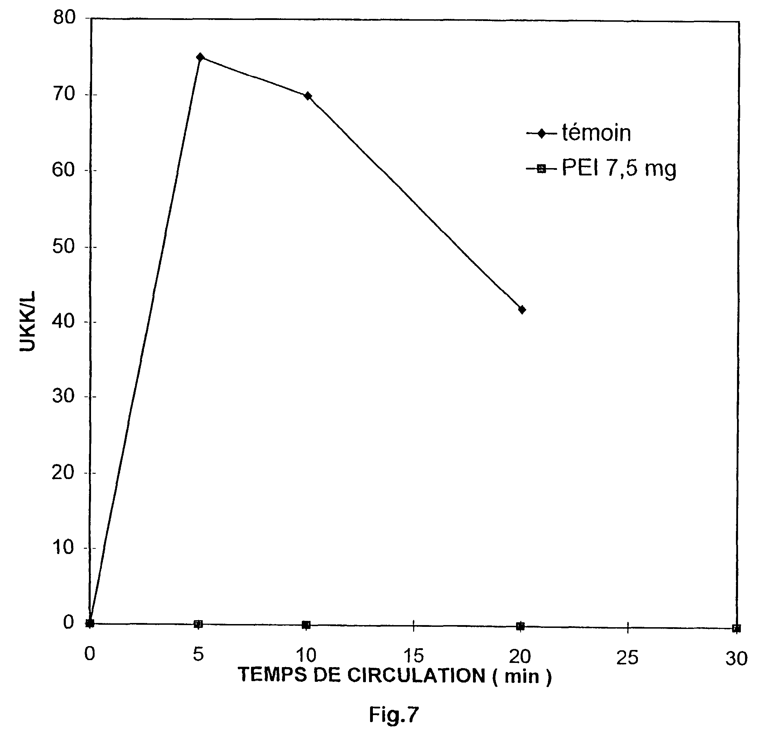

Le dialyseur de modèle PAN 13 DX a alors été soumis au test in vitro décrit dans l'exemple 1, destiné à mesurer si la membrane revêtue d'une monocouche de PEI active la phase contact. Le résultat du test a été négatif. La figure 7 représente le niveau d'activation causé par ce dialyseur et le niveau d'activation causé par un dialyseur de même fabrication mais dont la membrane n'a pas été revêtue de PEI.

### Exemple 10

On a vérifié la faisabilité de l'invention en utilisant, comme substance destinée à modifier une membrane, non plus une substance disponible sous forme de gel (comme la PEI), mais une substance disponible sous forme de poudre.

On a préparé un gel hydrosoluble non toxique et chimiquement neutre vis-à-vis de la membrane en AN69 en faisant dissoudre à température ambiante (25°C) du carboxyméthyl-cellulose (C 5678 de SIGMA) dans de l'eau déminéralisée à raison de 12,5% en masse de carboxyméthyl-cellulose et de 87,5% en masse d'eau. On a incorporé dans cette matrice neutre, comme substance active, du diéthylaminoéthyl dextran (D 1162 de SIGMA) de masse moléculaire moyenne 500 k µ, à raison de 50% en masse de DEAE dextran et de 50% en masse de gel de carboxyméthyl-cellulose.

Un dialyseur comprenant environ 8500 fibres creuses AN69 a été assemblé. Chaque fibre a les dimensions suivantes : diamètre interne : 240 µm ; épaisseur de la paroi : 50µm ; longueur : 0,225 m. La surface de la membrane destinée à venir au contact du sang est de environ 1,44 m². Une goutte de 100 mg du mélange gel neutre/substance active (soit 50 mg de DEAE dextran) a été déposée dans une tubulure d'accès au compartiment sang. Deux litres de sérum physiologique stérile (solution de chlorure de sodium à 0,9 g/l) à température ambiante (25°C) ont été mis en circulation à un débit de 200 ml/min dans le compartiment sang de ce dialyseur à partir de l'accès où la goutte de mélange gel neutre/substance active à été déposée. Le sérum physiologique a dissout la goutte de gel et les molécules de DEAE dextran mises en circulation dans le dialyseur se sont liées, par liaison ionique, aux groupes méthallyle sulfonate de sodium présents à la surface de la membrane.

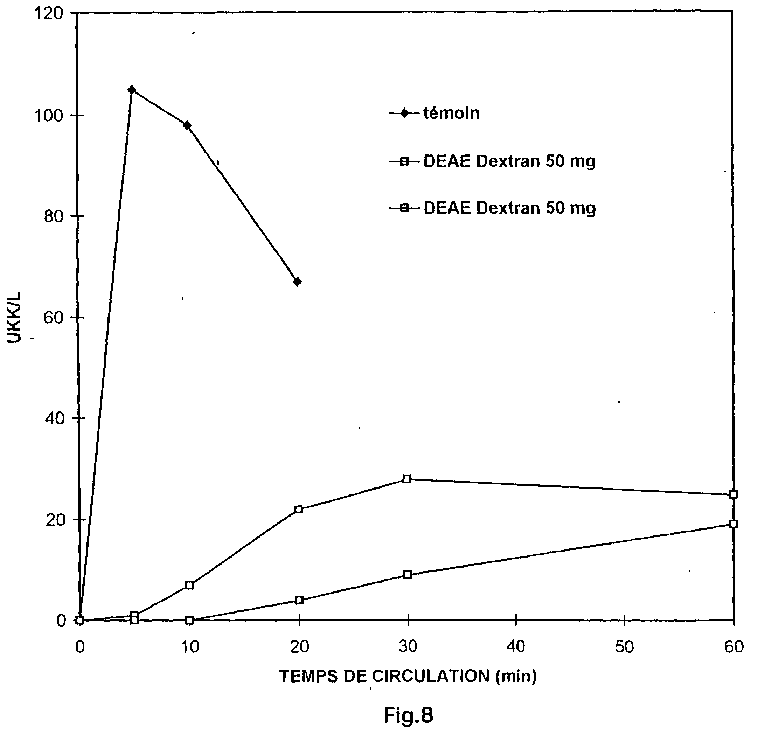

Pour mesurer si et, le cas échéant, dans quelle mesure le dialyseur ainsi fabriqué activait la phase contact, on l'a soumis au test décrit à l'exemple 1, à cette variante près que le plasma humain dilué à 5% dans du sérum physiologique a été mis en circulation dans le compartiment sang du dialyseur pendant une heure et non pas six, ce qui était suffisant compte tenu du but de la vérification effectuée. Le résultat des dosages montre sans équivoque que ce dialyseur ne provoque que faiblement l'activation de la phase contact (Figure 8). Il est à noter que, dans cet exemple, compte tenu de l'objectif visé, on n'a pas cherché à optimiser les paramètres permettant d'obtenir la neutralisation totale de la phase contact, en particulier la quantité de substance active et la viscosité du gel matriciel.

Pour finir, on a fait subir au dialyseur traité avec de la PEI toute la série de mesures classiques destinée à en déterminer les caractéristiques : toxicité, hémocompatibilité, capacité de transfert diffusif et convectif, etc. Les caractéristiques du dialyseur traité sont au moins aussi bonnes que celles d'un dialyseur de même type non traité.

### Test de toxicité

On a assemblé deux lots de dialyseurs ayant une membrane composée de fibres creuses en AN69, chaque fibre ayant les dimensions suivantes : diamètre interne : 210 µm; épaisseur de la paroi 42 µm. Les dialyseurs du premier lot comprenaient environ 9024 fibres (longueur : 0,24 m) et les dialyseurs du second lot comprenaient environ 11520 fibres (longueur : 0,30 m), la surface de la membrane destinée à venir au contact du sang étant de environ 1,43 m² dans le premier cas et de environ 2,26 m2 dans le second cas. Chaque dialyseur a ensuite été soumis à un traitement comprenant les étapes de:
1) déglycériner les fibres en rinçant le dialyseur avec de l'eau;
2) faire circuler pendant 6 min dans le compartiment sang, en circuit ouvert, à un débit de 200 ml/min, une solution aqueuse de PEI (LUPASOL WF; masse moléculaire: 25 k µ) à une concentration de 1 g/l. A la fin de cette étape, environ 340 mg de PEI sont fixés sur la membrane d'un dialyseur du premier lot (environ 9024 fibres) et environ 500 mg de PEI sont fixés sur la membrane d'un dialyseur du second lot (environ 11520 fibres) ;
3) stériliser le dialyseur par irradiation γ (36 kGy).

On note que la quantité de PEI fixée sur la membrane de ces dialyseurs était très supérieure à la quantité fixée sur la membrane des dialyseurs des exemples 1, 2 et 8 et que la stérilisation a été effectuée alors que la PEI était déjà liée à la membrane.

Ces dialyseurs ont satisfait aux essais d'évaluation biologique des dispositifs médicaux définis dans la norme internationale ISO 10993-1.

### Evaluation de l'hémocompatibilité

L'hémocompatibilité d'une membrane de dialyse, et en particulier son caractère non thrombogène, est liée à ses propriétés d'adsorption de protéines à la surface de la membrane en contact avec le sang.

La cinétique d'adsorption du fibrinogène marqué à l'iode 125 a été mesurée *in vitro* sur des dialyseurs de modèle réduit ayant une membrane composée de fibres en AN 69, dans des conditions hydrodynamiques comparables à celles d'une séance de dialyse.

Les dialyseurs de modèle réduit utilisés pour les essais comprenaient 170 fibres en AN 69 (diamètre interne : 210 µm; épaisseur de la paroi : 42 µm; longueur : 0,18 m), dont la surface destinée à venir au contact du sang avait été traitée avec de la PEI conformément à l'invention (environ 30 mg/m², respectivement 300 mg/m², pour les mini dialyseurs dans le compartiment sang desquels la goutte de PEI déposée était de 0,7 mg, respectivement de 7 mg).

On a préparé du sang humain hépariné contenant du fibrinogène radiomarqué à une concentration de 2,5 µg/ml et on a fait circuler ce liquide, en circuit ouvert, à un débit de 2,5 ml/min, dans un mini dialyseur témoin (AN 69 sans PEI) et dans un mini dialyseur selon l'invention. La vitesse d'adsorption du fibrinogène, déterminée au moyen de la mesure de la radioactivité du mini dialyseur, est représentée sur la figure 8b. On constate que cette vitesse est sensiblement la même pour un mini dialyseur témoin et pour les mini dialyseurs sur la membrane desquels de la PEI a été fixée.

Le même essai a été effectué avec une solution de fibrinogène radiomarqué dans un liquide tampon de pH 7,4 (concentration de 2,5 µg/ml). Comme on peut le constater sur la figure 8a, ici aussi, la vitesse d'adsorption est sensiblement la même pour un mini dialyseur témoin et pour un mini dialyseur sur la membrane duquel de la PEI a été fixée.

### Capacité de transfert convectif

On a mesuré la pente d'ultrafiltration et le débit d'ultrafiltration maximal de deux dialyseurs témoins (N°1 et 2) ayant une membrane plane en AN69 d'une surface de 1,53 m² et deux dialyseurs (N°3 et 4) ayant une membrane plane en AN69 d'une surface de 1,53 m², préparés selon le procédé décrit dans l'exemple 2 avec 12 mg de PEI (LUPASOL WF, de BASF; masse moléculaire : 25 k µ).

La mesure de la pente d'ultrafiltration et la mesure du débit d'ultrafiltration maximal ont été effectuées de la façon suivante: On fait circuler du sang de boeuf hépariné et standardisé (taux protéique de 60 g/l et hématocrite de 32 %) dans le compartiment sang du dialyseur au débit fixe de 300 ml/min. On provoque une ultrafiltration du sang au travers de la membrane au moyen d'une pompe connectée au second compartiment du dialyseur. En augmentant progressivement le débit d'ultrafiltration, on augmente la pression transmembranaire (PTM), qui est mesurée en continu à l'aide de deux capteurs de pression respectivement reliés aux deux compartiments du dialyseur, et on en déduit la pente d'ultrafiltration en ml/h.mmHg. A partir d'une valeur de seuil, le débit d'ultrafiltration reste stable même si la PTM continue d'augmenter. On mesure alors le débit d'ultrafiltration maximum en ml/min.

Le tableau ci-dessous indique le résultat de ces mesures, qui fait apparaître que les dialyseurs classiques et les dialyseurs selon l'invention ont des capacités de transfert convectif équivalentes.

| Dialyseur N° | Pente d'ultrafiltration (ml/h.mmHg) | Débit d'ultrafiltration maximal ml/min |
|---|---|---|
| 1 | 39 | 100 |
| 2 | 43 | 104 |
| 3 | 43 | 107 |
| 4 | 45 | 104 |

## Revendications

1. Procédé de fabrication d'un appareil pour le traitement du sang ou du plasma par circulation extracorporelle ayant un compartiment pour la circulation du sang muni de deux accès et d'une surface interne à traiter avec une substance, comprenant l'étape de :
- déposer dans le compartiment sang, au niveau d'au moins l'un des accès (9 ;10), une quantité déterminée (11,12) de la substance, la substance étant apte à se dissoudre dans une solution aqueuse et apte à se lier durablement à la surface interne à traiter du compartiment sang pour en accroître la biocompatibilité, la quantité de substance étant choisie pour que la circulation d'un volume déterminé de solution aqueuse dans le compartiment sang, à partir de l'accès où la substance a été déposée, ait pour résultat la formation par liaison durable d'au moins une couche moléculaire de la substance sur la surface interne à traiter du compartiment sang,
**caractérisé en ce que** :
- après que le dépôt de la substance a été effectué, et avant la circulation de la solution aqueuse, le procédé comprend l'étape de stériliser l'appareil, la quantité déterminée (11,12) de substance étant déposée sous une forme telle que la substance puisse subir sans altération une irradiation propre à stériliser l'appareil.

2. Procédé selon la revendication 1, pour un appareil dont le compartiment sang a deux accès symétriques (9, 10), **caractérisé en ce que** la quantité déterminée de substance est déposée au niveau de chaque accès de façon que, quel que soit le sens de circulation ultérieure de la solution aqueuse dans le compartiment sang, une couche moléculaire de la substance soit formée sur la surface interne à traiter.

3. Procédé selon une des revendications 1 à 2, **caractérisé en ce que** la quantité déterminée (11,12) de substance est déposée sous la forme d'une goutte d'un gel de cette substance.

4. Procédé selon une des revendications 1 ou 2, **caractérisé en ce que** la quantité déterminée (11,12) de substance est déposée sous la forme d'une goutte d'un matériau matrice soluble dans une solution aqueuse, dans lequel la substance est incorporée.

5. Procédé selon la revendication 1, **caractérisé en ce que** l'appareil est stérilisé au moyen d'oxyde d'éthylène.

6. Procédé selon la revendication 1, **caractérisé en ce que** l'appareil est stérilisé par irradiation γ.

7. Procédé selon la revendication 1, **caractérisé en ce que** la quantité déterminée (11,12) de substance est déposée après que l'appareil a été assemblé.

8. Appareil pour le traitement du sang ou du plasma par circulation extracorporelle comprenant :
- un compartiment pour la circulation du sang muni de deux accès (9 ; 10);
- une quantité déterminée (11 ; 12) d'une substance soluble dans une solution aqueuse et déposée au niveau d'au moins l'un des accès (9 ; 10) dans le compartiment sang ;
- le compartiment ayant au moins une surface interne destinée à être revêtue d'au moins une couche moléculaire de ladite substance ;
- l'appareil pour le traitement, avant la circulation de la solution aqueuse, muni de la substance déposée est un appareil stérilisé.
- la substance déposée est apte à se lier à la surface interne à traiter du compartiment sang pour en accroître la biocompatibilité ;
- la quantité de substance est choisie pour que la circulation d'un volume déterminé de solution aqueuse dans le compartiment sang à partir de l'accès où la substance a été déposée ait pour résultat la formation par liaison durable de ladite couche moléculaire de la substance sur la surface interne à traiter du compartiment sang, **caractérisé en ce que** :
- la forme de la quantité déterminée de la substance déposée est telle que la substance puisse subir sans altération une irradiation propre à la stérilisation de l'appareil; et

9. Appareil selon la revendication 8, ayant deux accès symétriques, **caractérisé en ce que** la quantité déterminée (11,12) de substance est déposée au niveau de chaque accès de façon que, quel que soit le sens de la circulation ultérieure de la solution aqueuse dans le compartiment sang, une couche moléculaire de la substance soit formée sur la surface interne à traiter.

10. Appareil selon une des revendications 8 et 9, **caractérisé en ce que** la quantité déterminée (11,12) de substance se présente sous la forme d'une goutte d'un gel de cette substance.

11. Appareil selon une des revendications 8 et 9, **caractérisé en ce que** la quantité déterminée (11,12) de substance se présente sous la forme d'une goutte d'un matériau matrice dans lequel la substance est incorporée.

12. Appareil selon une des revendications 8 à 11, **caractérisé en ce que** la surface à traiter présente des charges négatives et **en ce que** la substance est cationique de sorte qu'une couche moléculaire de la substance masque les charges négatives de la surface.

13. Appareil selon une des revendications 8 à 12, constitué par un hémodialyseur/hémofiltre comprenant deux compartiments séparés par une membrane semi perméable, un premier compartiment étant destiné à la circulation de sang et un second compartiment étant destiné à la circulation de liquide. usé, **caractérisé en ce que** la surface à traiter est la surface de la membrane semi perméable située du côté du compartiment sang.

14. Appareil selon la revendication 13, **caractérisé en ce que** la membrane est fabriquée à partir d'un copolymère d'acrylonitrile et **en ce que** la substance est de la polyéthylèneimine.

15. Appareil selon la revendication 14, **caractérisé en ce que** la polyéthylèneimine a une masse moléculaire comprise entre environ 10 et environ 2000 k u.

16. Appareil selon la revendication 15, **caractérisé en ce que** la polyéthylèneimine a une masse moléculaire de 25 k u.

17. Appareil selon la revendication 15, **caractérisé en ce que** la polyéthylèneimine a une masse moléculaire de 750 k u.

18. Appareil selon la revendication 15, **caractérisé en ce que** la quantité de polyéthylèneimine de masse moléculaire de 25 k u déposée au niveau d'un ou de chaque accès sang est comprise entre environ 5 mg et environ 10 mg par m² de membrane active.

19. Appareil selon une des revendications 13 à 18, **caractérisé en ce que** la membrane est une membrane plane.

20. Appareil selon une des revendications 13 à 18, **caractérisé en ce que** la membrane est constituée par un faisceau de fibres creuses.

## Patentansprüche

1. Verfahren zur Herstellung einer Vorrichtung zur Blut- oder Plasmabehandlung durch extrakorporale Zirkulation mit einem Abteil zum Blutzirkulieren, der zwei Eingänge und eine mit einem Stoff zu behandelnde innere Oberfläche aufweist, umfassend den folgenden Schritt:
- Ablagern im Blutabteil an mindestens einem der Eingänge (9; 10) von einer vorgegebenen Menge (11, 12) des Stoffes, wobei der Stoff in einer wässrigen Lösung gelöst werden kann und sich dauerhaft mit der zu behandelnden inneren Oberfläche des Blutabteils binden kann, um deren Biokompatibilität zu erhöhen, wobei die Stoffmenge dazu gewählt wird, damit die Zirkulation eines vorgegebenen Volumens von wässriger Lösung im Blutabteil von dem Eingang, wo der Stoff abgelagert worden ist, zur Bildung durch dauerhafte Bindung von mindestens einer Molekularschicht des Stoffes an der zu behandelnden inneren Oberfläche des Blutabteiles führt,
**dadurch gekennzeichnet, daß**:
- nach der Ablagerung des Stoffes and vor der Zirkulation der wässrigen Lösung umfasst das Verfahren einen Schritt, bei dem die Vorrichtung sterilisiert wird, wobei die vorgegebene Stoffmenge (11, 12) in einer solchen Form abgelagert wird, daß der Stoff einer Strahlung zum Sterilisieren der Vorrichtung ohne Veränderung unterworfen werden kann.

2. Verfahren nach Anspruch 1, für eine Vorrichtung, deren Blutabteil zwei symmetrische Eingänge (9, 10) aufweist, **dadurch gekennzeichnet, daß** die vorgegebene Stoffmenge an jedem Eingang so abgelagert wird, daß unabhängig von der Richtung der weiteren Zirkulation der wässrigen Lösung im Blutabteil eine Molekularschicht des Stoffes an der zu behandelnden inneren Oberfläche gebildet wird.

3. Verfahren nach einem der Ansprüche 1 bis 2, **dadurch gekennzeichnet, daß** die vorgegebene Stoffmenge (11, 12) in der Form eines Tropfes eines Gels von diesem Stoff abgelagert wird.

4. Verfahren nach einem der Ansprüche 1 oder 2, **dadurch gekennzeichnet, daß** die vorgegebene Stoffmenge (11, 12) in der Form eines Tropfes eines in einer wässrigen Lösung löslichen Matrixmaterials, worin das Stoff eingebettet ist.

5. Verfahren nach Anspruch 1, **dadurch gekennzeichnet, daß** die Vorrichtung durch Ethylenoxyd sterilisiert wird.

6. Verfahren nach Anspruch 1, **dadurch gekennzeichnet, daß** die Vorrichtung durch γ-Strahlung sterilisiert wird.

7. Verfahren nach Anspruch 1, **dadurch gekennzeichnet, daß** die vorgegebene Stoffmenge (11, 12) nach der Montage der Vorrichtung abgelagert wird.

8. Vorrichtung zur Blut- oder Plasmabehandlung durch extrakorporale Zirkulation, umfassend:
- einen Abteil zum Blutzirkulieren, der zwei Eingänge (9; 10) aufweist;
- eine vorgegebene Menge (11; 12) eines Stoffes, das in einer wässrigen Lösung löslich ist und an mindestens einem der Eingänge (9; 10) im Blutabteil abgelagert wird;
- wobei der Abteil mindestens eine innere Oberfläche aufweist, die mit mindestens einer Molekularschicht des Stoffes verkleidet werden soll;
- wobei das abgelagerte Stoff sich mit der zu behandelnden inneren Oberfläche des Blutabteils binden kann, um deren Biokompatibilität zu erhöhen;
- wobei die Stoffmenge so gewählt wird, damit die Zirkulation eines vorgegebenen Volumens von wässriger Lösung im Blutabteil von dem Eingang, wo der Stoff abgelagert worden ist, zur Bildung durch dauerhafte Bindung der Molekularschicht des Stoffes an der zu behandelnden inneren Oberfläche des Blutabteiles führt,
**dadurch gekennzeichnet, daß**:
- die Form der abgelagerten vorgegebenen Stoffmenge ist solche, daß der Stoff einer Strahlung zum Sterilisieren der Vorrichtung ohne Veränderung unterworfen werden kann, und
- die Behandlungsvorrichtung mit dem abgelagerten Stoff vor der Zirkulation der wässrigen Lösung eine sterilisierte Vorrichtung ist.

9. Vorrichtung nach Anspruch 8, mit zwei symmetrischen Eingängen, **dadurch gekennzeichnet, daß** die vorgegebene Stoffmenge (11, 12) an jedem Eingang so abgelagert wird, daß unabhängig von der Richtung der weiteren Zirkulation der wässrigen Lösung im Blutabteil eine Molekularschicht des Stoffes an der zu behandelnden inneren Oberfläche gebildet wird.

10. Vorrichtung nach einem der Ansprüche 8 und 9, **dadurch gekennzeichnet, daß** die vorgegebene Stoffmenge (11, 12) in der Form eines Tropfes eines Gels von diesem Stoff vorliegt.

11. Vorrichtung nach einem der Ansprüche 8 und 9, **dadurch gekennzeichnet, daß** die vorgegebene Stoffmenge (11, 12) in der Form eines Tropfes eines Matrixmaterials vorliegt, worin das Stoff eingebettet ist.

12. Vorrichtung nach einem der Ansprüche 8 bis 11, **dadurch gekennzeichnet, daß** die zu behandelnde Oberfläche negative Ladungen aufweist, und daß das Stoff kationisch ist, so daß eine Molekularschicht des Stoffes die negativen Ladungen der Oberfläche verkleidet.

13. Vorrichtung nach einem der Ansprüche 8 bis 12, die aus einem Hämodialysator/Hämofilter besteht, der zwei durch eine semipermeable Membran getrennten Abteile umfasst, wobei ein erster Abteil zum Blutzirkulieren dient und ein zweiter Abteil zum Zirkulieren von gebrauchter Flüssigkeit dient, **dadurch gekennzeichnet, daß** die zu behandelnde Oberfläche die Oberfläche der an der Seite des Blutabteiles liegenden semipermeablen Membran ist.

14. Vorrichtung nach Anspruch 13, **dadurch gekennzeichnet, daß** die Membran ausgehend von einem Acrylnitryl-Copolymer hergestellt wird, und daß das Stoff Polyethylenimin ist.

15. Vorrichtung nach Anspruch 14, **dadurch gekennzeichnet, daß** Polyethylenimin eine Molekularmasse von ca. 10 bis ca. 2000 k u hat.

16. Vorrichtung nach Anspruch 15, **dadurch gekennzeichnet, daß** Polyethylenimin eine Molekularmasse von 25 k u hat.

17. Vorrichtung nach Anspruch 15, **dadurch gekennzeichnet, daß** Polyethylenimin eine Molekularmasse von 750 k u hat.

18. Vorrichtung nach Anspruch 15, **dadurch gekennzeichnet, daß** die Menge von Polyethylenimin mit einer Molekularmasse von 25 k u, die an einem oder jedem der Bluteingänge abgelagert wird, von ca. 5 mg bis ca. 10 mg pro m² von aktiver Membran ist.

19. Vorrichtung nach einem der Ansprüche 13 bis 18, **dadurch gekennzeichnet, daß** die Membran eine flache Membran ist.

20. Vorrichtung nach einem der Ansprüche 13 bis 18, **dadurch gekennzeichnet, daß** die Membran aus einem Bündel von Hohlfasern besteht.

## Claims

1. A method for making an equipment for blood or plasma treatment by means of extracorporeal circulation, having a compartment for blood circulation provided with two inlets and with an inner surface to be treated with a substance, comprising the following step:
- laying in the blood compartment, at least at one of the inlets (9; 10), a given amount (11, 12) of substance, said substance being able to dissolve in an aqueous solution and being able to bind permanently with the inner surface to be treated of the blood compartment so as to increase its biocompatibility, the amount of substance being chosen so that the circulation of a given volume of aqueous solution in the blood compartment, starting from the inlet where the substance has been laid, results in the formation through a permanent bond of at least one molecular layer of substance on the inner surface to be treated of the blood compartment,
**characterized in that**:
- after laying the substance and before the circulation of the aqueous solution, the method comprises the step of sterilizing the equipment, the given amount (11, 12) of substance being laid in such a form that the substance can undergo without changing irradiation for equipment sterilization.

2. The method according to claim 1, for an equipment whose blood compartment has two symmetric inlets (9, 10), **characterized in that** the given amount of substance is laid at each inlet so that, whatever the direction of further circulation of the aqueous solution in the blood compartment, a molecular layer of substance is formed on the inner surface to be treated.

3. The method according to one of the claims 1 to 2, **characterized in that** the given amount (11, 12) of substance is laid as a drop of gel of said substance.

4. The method according to one of the claims 1 or 2, **characterized in that** the given amount (11, 12) of substance is laid as a drop of matrix material, soluble in an aqueous solution, into which the substance is incorporated.

5. The method according to claim 1, **characterized in that** the equipment is sterilized with ethylene oxide.

6. The method according to claim 1, **characterized in that** the equipment is sterilized with γ irradiation.

7. The method according to claim 1, **characterized in that** the given amount (11, 12) of substance is laid after assembling the equipment

8. An equipment for blood or plasma treatment by means of extracorporeal circulation, comprising:
- a compartment for blood circulation provided with two inlets (9; 10);
- a given amount (11; 12) of a substance soluble in an aqueous solution and laid at least at one of the inlets (9; 10) in the blood compartment;
- the compartment having at least one inner surface designed to be coated with at least one molecular layer of said substance;
- the laid substance is able to bind with the inner surface to be treated of the blood compartment so as to increase its biocompatibility;
- the amount of substance is chosen so that the circulation of a given volume of aqueous solution in the blood compartment, starting from the inlet where the substance has been laid, results in the formation through a permanent bond of said molecular layer of substance on the inner surface to be treated of the blood compartment,
**characterized in that**:
- the form of the given amount of laid substance is such that the substance can undergo without changing irradiation for equipment sterilization, and
- the treatment equipment, before the circulation of the aqueous solution, provided with the laid substance, is a sterilized equipment.

9. The equipment according to claim 8, having two symmetric inlets, **characterized in that** the given amount (11, 12) of substance is laid at each inlet so that, whatever the direction of further circulation of the aqueous solution in the blood compartment, a molecular layer of substance is formed on the inner surface to be treated.

10. The equipment according to one of the claims 8 and 9, **characterized in that** the given amount (11, 12) of substance has the form of a drop of gel of said substance.

11. The equipment according to one of the claims 8 and 9, **characterized in that** the given amount (11, 12) of substance has the form of a drop of matrix material into which the substance is incorporated.

12. The equipment according to one of the claims 8 to 11, **characterized in that** the surface to be treated has negative charges and **in that** the substance is cationic so that a molecular layer of substance covers the negative charges of the surface.

13. The equipment according to one of the claims 8 to 12, made up of a hemo-dialyzer/hemofilter comprising two compartments separated by a semi-permeable membrane, a first compartment being designed for blood circulation and a second compartment being designed for the circulation of used liquid, **characterized in that** the surface to be treated is the surface of the semi-permeable membrane placed on the side of the blood compartment.

14. The equipment according to claim 13, **characterized in that** the membrane is obtained from an acrylonitrile copolymer and **in that** the substance is polyethylene imine.

15. The equipment according to claim 14, **characterized in that** polyethylene imine has a molecular mass of about 10 to about 2000 k u.

16. The equipment according to claim 15, **characterized in that** polyethylene imine has a molecular mass of 25 k u.

17. The equipment according to claim 15, **characterized in that** polyethylene imine has a molecular mass of 750 k u.

18. The equipment according to claim 15, **characterized in that** the amount of polyethylene imine having a molecular mass of 25 k u laid at one or at each of the blood inlets is of about 5 mg to about 10 mg for m² of active membrane.

19. The equipment according to one of the claims 13 to 18, **characterized in that** the membrane is a flat membrane.

20. The equipment according to one of the claims 13 to 18, **characterized in that** the membrane is made up of a bundle of hollow fibers.
